## Europäisches Patentamt
## European Patent Office
## Office européen des brevets

(11) Veröffentlichungsnummer: **0 055 361**
A1

(12)
# EUROPÄISCHE PATENTANMELDUNG

(21) Anmeldenummer: 81108882.2

(22) Anmeldetag: 24.10.81

(51) Int. Cl.³: **C 07 C 11/167**
C 07 C 7/148, C 07 C 7/08
C 07 C 41/06, C 07 C 41/42
C 07 C 43/04

(30) Priorität: 27.12.80 DE 3049213

(43) Veröffentlichungstag der Anmeldung:
07.07.82 Patentblatt 82/27

(84) Benannte Vertragsstaaten:
AT BE DE FR GB IT NL

(71) Anmelder: BASF Aktiengesellschaft
Carl-Bosch-Strasse 38
D-6700 Ludwigshafen(DE)

(72) Erfinder: Lindner, Alfred, Dr.
Ringstrasse 30
D-6712 Bobenheim-Roxheim(DE)

(72) Erfinder: Volkamer, Klaus, Dr.
Heidelberger Ring 21
D-6710 Frankenthal(DE)

(72) Erfinder: Wagner, Ulrich, Dr.
Knospstrasse 7
D-6703 Limburgerhof(DE)

(72) Erfinder: Sandrock, Gerhard, Dr.
Albrecht-Duerer-Ring 36C
D-6710 Frankenthal(DE)

(72) Erfinder: Strohmeyer, Max, Dr.
Woogstrasse 41
D-6703 Limburgerhof(DE)

(54) Verfahren zur gemeinsamen Herstellung von Alkyl-tert.-butylether und Gewinnung von Butadien.

(57) Die vorliegende Erfindung betrifft ein Verfahren zur gemeinsamen Herstellung von Alkyl-tert.-butylether und Gewinnung von Butadien mit Hilfe eines selektiven Lösungsmittels aus einem $C_4$-Kohlenwasserstoffgemisch, welches Butadien, Isobuten, im selektiven Lösungsmittel leichter als Butadien lösliche Kohlenwasserstoffe und im selektiven Lösungsmittel schwerer als Butadien lösliche Kohlenwasserstoffe enthält, bei dem man

a) das $C_4$-Kohlenwasserstoffgemisch in Gegenwart eines sauren Katalysators mit einem primären $C_1$- bis $C_6$-Alkohol reagieren läßt, wobei das Isobuten ganz oder teilweise zum Alkyl-tert.-butylether umgesetzt wird, und

b) das erhaltene Reaktionsgemisch anschließend durch ein- oder mehrstufige Extraktivdestillation in
- einen die im selektiven Lösungsmittel schwerer als Butadien löslichen Kohlenwasserstoffe enthaltenden Strom,
- einen das Butadien enthaltenden Strom und
- einen den gebildeten Alkyl-tert.-butylether und die im selektiven Lösungsmittel leichter als Butadien löslichen Kohlenwasserstoffe enthaltenden Strom auftrennt.

EP 0 055 361 A1

BASF Aktiengesellschaft                    O.Z. 0050/034840

Verfahren zur gemeinsamen Herstellung von
Alkyl-tert.-butylether und Gewinnung von Butadien

Die Erfindung betrifft ein Verfahren zur gemeinsamen Herstellung von Alkyl-tert.-butylether und Gewinnung von Butadien mit Hilfe eines selektiven Lösungsmittels aus einem Isobuten und Butadien enthaltenden $C_4$-Kohlenwasserstoffgemisch.

Es ist bereits bekannt, z.B. aus der DE-AS 25 21 965 (US-Patentschrift 4 020 114), Butadien aus einem $C_4$-Kohlenwasserstoffgemisch, das Butadien, Isobuten und Acetylenverbindungen enthält, in der Weise zu gewinnen, daß zunächst das Isobuten in Anwesenheit eines sauren Ionenaustauscherharzes mit Methanol verethert wird und der erhaltene Methyl-tert.-butylether aus dem Reaktionsgemisch durch Destillation von den nicht umgesetzten $C_4$-Kohlenwasserstoffen abgetrennt wird. Die in dem Gemisch der nicht umgesetzten $C_4$-Kohlenwasserstoffe enthaltenen Acetylenverbindungen werden anschließend in einem zweiten Reaktor in Anwesenheit eines sauren Ionenaustauscherharzes, dem Quecksilberionen zugefügt worden sind, mit zusätzlich zugesetztem Methanol unter Bildung der Vinylether verethert. Das in dem zweiten Reaktor erhaltene Reaktionsgemisch wird anschließend durch extraktive Destillation in einen die Butane und Butene enthaltenden Strom, einen Butadienstrom und einen die Vinylether enthaltenden Strom aufgetrennt. Dieses Verfahren hat jedoch den Nachteil, daß die Acetylenverbindungen zum Zwecke der Abtrennung durch eine chemische Reaktion umgewandelt werden müssen und somit als Wertstoffe verloren gehen. Weiter muß die Umsetzung der Acetylene in Gegenwart von Quecksilberverbindungen enthaltenden Ionenaustauschern durchgeführt werden, so daß das Verfahren wegen Ste/P

der Toxität von Quecksilberverbindungen mit erheblichen Umweltschutzproblemen verbunden ist.

Der Erfindung lag daher die Aufgabe zugrunde, ein Verfahren zur gemeinsamen Herstellung von Alkyl-tert.-butylether und Gewinnung von Butadien aus einem $C_4$-Kohlenwasserstoffgemisch zur Verfügung zu stellen, welches die Nachteile des bekannten Verfahrens nicht aufweist.

Es wurde nun ein vorteilhaftes Verfahren gefunden zur gemeinsamen Herstellung von Alkyl-tert.-butylether und Gewinnung von Butadien mit Hilfe eines selektiven Lösungsmittels aus einem $C_4$-Kohlenwasserstoffgemisch, welches Butadien, Isobuten, im selektiven Lösungsmittel leichter als Butadien lösliche Kohlenwasserstoffe und im selektiven Lösungsmittel schwerer als Butadien lösliche Kohlenwasserstoffe enthält, welches dadurch gekennzeichnet ist, daß man

a) das $C_4$-Kohlenwasserstoffgemisch in Gegenwart eines sauren Katalysators mit einem primären $C_1$- bis $C_6$-Alkohol reagieren läßt, wobei das Isobuten ganz oder teilweise zum Alkyl-tert.-butylether umgesetzt wird, und

b) das erhaltene Reaktionsgemisch anschließend durch ein- oder mehrstufige Extraktivdestillation in
- einen die im selektiven Lösungsmittel schwerer als Butadien löslichen Kohlenwasserstoffe enthaltenden Strom,
- einen das Butadien enthaltenden Strom und
- einen den gebildeten Alkyl-tert.-butylether und die im selektiven Lösungsmittel leichter als Butadien löslichen Kohlenwasserstoffe enthaltenden Strom auftrennt.

Nach dem neuen Verfahren ist es nicht erforderlich, zur Abtrennung der Acetylenverbindungen diese unter Verwendung von toxischen Quecksilberverbindungen in Vinylether umzuwandeln. Vielmehr wird ein die Acetylenverbindungen enthaltender Strom abgetrennt, in dem die $C_4$-Acetylene, z.B. das Vinylacetylen, chemisch unverändert vorhanden sind. Somit besteht beispielsweise die Möglichkeit, durch selektive katalytische Hydrierung des $C_4$-Acetylenstroms aus dem Vinylacetylen zusätzlich wertvolles Butadien zu gewinnen oder den $C_4$-Acetylenstrom im etherischen Lösungsmittel für chemische Reaktionen, z.B. als CH-acide Verbindungen, einzusetzen.

Als selektives Lösungsmittel kommen für das erfindungsgemäße Verfahren beispielsweise die für die Gewinnung von Butadien aus $C_4$-Kohlenwasserstoffgemischen geeigneten Lösungsmittel in Betracht. Vorzugsweise wird Acetonitril, Methoxyproprionitril, Butyrolacton, N-Formylmorpholin oder Furfurol, insbesondere ein N-alkylsubstituiertes niederes aliphatisches Säureamid oder ein N-alkylsubstituiertes alicyclisches Säureamid mit 5 Ringgliedern oder ihre Mischungen mit Wasser verwendet.

Geeignete N-alkylsubstituierte niedere aliphatische Säureamide sind z.B. Dimethylformamid, Diethylformamid, Dimethylacetamid, Diethylacetamid, Formylmorpholin. Als N-alkylsubstituierte alicyclische Säureamide (Lactame) kommen z.B. N-Alkylpyrrolidone, insbesondere N-Methylpyrrolidon, in Betracht. Im allgemeinen werden N-alkylsubstituierte niedere aliphatische Säureamide oder N-alkylsubstituierte alicyclische Säureamide mit Siedepunkten im Bereich von 150 bis 260°C, vorzugsweise im Bereich von 150 bis 210°C, verwendet. Mit besonderem Vorteil werden Dimethylformamid und insbesondere N-Methylpyrrolidon verwendet.

Bei einer bevorzugten Ausführungsform des erfindungsgemäßen Verfahrens enthält das in der extraktiven Destillation verwendete selektive Lösungsmittel 1 bis 90 Gew.%, vorzugsweise 2 bis 50 Gew.%, insbesondere 5 bis 30 Gew.%, gebildeten Alkyl-tert.-butylether, bezogen auf die Mischung aus selektivem Lösungsmittel und gebildetem Alkyl-tert.-butylether.

Die Verwendung der Mischung aus selektivem Lösungsmittel und gebildetem Alkyl-tert.-butylether als Lösungsmittel in der Extraktivdestillation hat den Vorteil, daß die Abtrennung der im selektiven Lösungsmittel gelösten Kohlenwasserstoffe vom selektiven Lösungsmittel in der Lösungsmittelwiedergewinnungszone, z.B. in einem Ausgaser, erleichtert wird. Durch den Ersatz von Wasser, welches in den bekannten Verfahren zur Butadienextraktion den selektiven Lösungsmitteln zur Selektivitätserhöhung zugesetzt wird, durch gebildeten Alkyl-tert.-butylether wird die Löslichkeit der $C_4$-Kohlenwasserstoffe in dem Lösungsmittel für die Extraktivdestillation bei annähernd gleicher Selektivität wesentlich erhöht, so daß die Menge an umlaufendem Lösungsmittel in der Butadienextraktionsanlage stark erniedrigt werden kann. Die Verwendung der Mischung aus selektivem Lösungsmittel und gebildetem Alkyl-tert.-butylether hat weiter den Vorteil, daß ein Teil des zu trennenden Gemisches als Colösungsmittel wirkt. Die Menge an zu trennendem Kohlenwasserstoffgemisch ist somit erheblich reduziert, wodurch die Menge an umlaufendem Lösungsmittel weiter reduziert werden kann. Dies hat insbesondere eine Senkung der Investitionskosten für die Extraktionsanlage und der Verbräuche an Dampf und elektrischer Energie zur Folge. Auf einen Wasserzusatz wie bei den bekannten Verfahren zur Verbesserung der Selektivität oder der Ausgasung kann verzichtet werden.

Bei Verwendung eines relativ hoch siedenden selektiven Lösungsmittels, z.B. ein Lösungsmittel mit einem Siedepunkt im Bereich von 140 bis 260°C, wird durch Verwendung der Mischung des selektiven Lösungsmittels mit einem niedriger als das selektive Lösungsmittel siedenden Alkyl-tert.-butylether, z.B. ein Alkylether mit einem Siedepunkt von höchstens 125°C, in der Extraktivdestillation zusätzlich der Vorteil erhalten, daß die beispielsweise als Ausgaser oder als Lösungsmittelabstreifer betriebene Lösungsmittelwiedergewinnungszone der Butadienextraktionsanlage bei jeweils gleicher Sumpftemperatur bei höherem Druck als mit den reinen selektiven Lösungsmitteln, d.h. ohne Zumischung von Alkyl-tert.-butylether, betrieben werden kann. Dies hat beispielsweise den Vorteil, daß die in der Lösungsmittelgewinnungszone erhaltenen ausgegasten Kohlenwasserstoffe aufgrund des höheren Drucks ohne Zwischenschaltung eines Kompressors in nachgeschaltete, unter erhöhtem Druck betriebenen Zonen gefördert werden können. Ein anderer Vorteil der Verwendung einer Mischung eines der relativ hoch siedenden selektiven Lösungsmittel mit einem niedriger als dieses siedenden Alkyl-tert.-butylether besteht darin, daß die z.B. als Ausgaser oder als Lösungsmittelabstreifer betriebene Lösungsmittelwiedergewinnungszone der Extraktionsanlage bei Anwendung des gleichen Drucks im Vergleich zur Verwendung des reinen selektiven Lösungsmittels bei einer niedrigeren Sumpftemperatur betrieben werden kann, so daß eine Verschmutzung der Extraktionsanlage durch Polymerbildung leichter vermieden werden kann.

Das erfindungsgemäße Verfahren weist weiter noch die folgenden Vorteile auf:

Selbst bei Anwendung von relativ niedrigen Ausgasungsdrücken in der Lösungsmittelwiedergewinnungszone,
z.B. von 1,5 bar, können die aus der Lösungsmittelwiedergewinnungszone abgezogenen $C_4$-Kohlenwasser-
stoffströme, z.B. der die $C_4$-Acetylene enthaltende
Strom, durch einfache Kühlung mit Wasser ohne Anwendung eines Kältemittels, z.B. Sole oder Propylen-
-Kühlung, in flüssiger Form erhalten werden.

-   In dem aus der Lösungsmittelwiedergewinnungszone abge-
    zogenen, die $C_4$-Acetylene und gleichzeitig den gebildeten Alkyl-tert.-butylether enthaltenden Strom,
    werden die $C_4$-Acetylene durch die Verdünnung mit den
    Alkyl-tert.-butylether mit niedrigen Partialdrücken
    und somit in ungefährlicher Form erhalten. Eine Ver-
    dünnung des $C_4$-Acetylenstroms mittels Wasser oder
    anderer Lösungsmittel mit dem Ziele, den Partialdruck
    der $C_4$-Acetylene zu senken, ist daher nicht erforderlich.

-   Trotz der Koppelung der Herstellung von Alkyl-tert.-
    -butylether und Gewinnung von Butadien weist das
    Gesamtverfahren überraschenderweise ein hohes Maß
    an Flexibilität auf.

-   Trotz der Mitverwendung des selektiven Lösungsmittels
    wird überraschenderweise der Trennaufwand in Bezug auf
    die Herstellung des Alkyl-tert.-butylethers im Vergleich zu den bekannten Verfahren vermindert.

-   Für eine eventuelle Weiterverarbeitung der im er-
    findungsgemäßen Verfahren abgetrennten $C_4$-Acetylene,
    insbesondere des Vinylacetylens, z.B. durch selektive
    katalytische Hydrierung des Vinylacetylens zu Butadien, ist es von Vorteil, daß das Vinylacetylen in flüssiger, relativ reiner Form in dem gebildeten Alkyl-tert.-butylether gelöst, d.h. in Lösung in einem billigen, niedrig siedenden etherischen Lösungsmittel, erhalten wird.

Für das erfindungsgemäße Verfahren geeignete, Butadien und Isobuten enthaltende $C_4$-Kohlenwasserstoffgemische werden z.B. als $C_4$-Fraktionen bei der Herstellung von Ethylen und/oder Propylen durch thermisches Spalten einer Petroleumfraktion, z.B. von verflüssigtem Petroleumgas (LPG), Leichtbenzin (Naphtha), Gasöl oder dgl. als Kohlenwasserstoff-Fraktion erhalten. Weiterhin werden solche $C_4$-Fraktionen bei der katalytischen Dehydrierung von n-Butan und/oder n-Buten erhalten. Die $C_4$-Fraktionen enthalten in der Regel Butane, n-Buten, Isobuten, Butadien-1,3, Vinylacetylen, Butin-1, Butadien-1,2 und gegebenenfalls geringe Mengen an $C_5$-Kohlenwasserstoffen, wobei der Butadiengehalt im allgemeinen 10 bis 80 Gew.%, vorzugsweise 20 bis 70 Gew.%, insbesondere 30 bis 60 Gew.% beträgt, der Isobutylengehalt im allgemeinen 10 bis 60 Gew.%, vorzugsweise 15 bis 50 Gew.% beträgt, während der Gehalt der $C_4$-Fraktionen an Vinylacetylen, Butin-1 und Butadien-1,2 insgesamt 5 Gew.% im allgemeinen nicht übersteigt.

Bei der Extraktivdestillation dieser $C_4$-Fraktionen sind im allgemeinen die gesättigten und einfach olefinisch ungesättigten $C_4$-Kohlenwasserstoffe wie Butane und Butene im selektiven Lösungsmittel schwerer als Butadien löslich, während die im selektiven leichter löslichen Kohlenwasserstoffe Vinylacetylen, Butin-1 und Butadien-1,2 einschließen.

Für die Umsetzung des $C_4$-Kohlenwasserstoffgemisches in Gegenwart eines sauren Katalysators mit einem primären $C_1$- bis $C_6$-Alkohol in der Veretherungsstufe, wobei das Isobuten ganz oder teilweise zum Alkyl-tert.-butylether um-

gesetzt wird, werden als primäre $C_1$- bis $C_6$-Alkohole beispielsweise Methanol, Ethanol, n-Propanol, Isobutanol, n-Butanol, n-Pentanol, Isopentanol, n-Hexylalkohol verwendet. Die Alkohole werden z.B. als technische Produkte üblicher Reinheit, beispielsweise mit einer Reinheit von mindestens 95 %, vorzugsweise 98 % verwendet.

Als saure Kondensationsmittel für die Veretherung kommen beispielsweise Mineralsäuren wie Schwefelsäure, Phosphorsäure, organische Sulfonsäuren wie Benzolsulfonsäure, p-Toluolsulfonsäure, saure Aluminiumsalze oder saure Katalysatoren vom Friedel-Crafts-Typ wie Kupfer(II)-chlorid, Eisen(II)-chlorid sowie vorzugsweise Ionenaustauscher in der Wasserstofform in Betracht. Geeignete Ionenaustauscher sind beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte Polystyrol-Harze wie Kern-sulfonierte vernetzte Styrol-Divinylbenzol-Copolymerisate. Bei Verwendung eines flüssigen oder gelösten sauren Kondensationsmittels beträgt die Menge des flüssigen oder gelösten sauren Kondensationsmittels im allgemeinen etwa 0,001 bis 0,9 l, vorzugsweise 0,01 bis 0,7 l pro 1 Reaktorvolumen. Bei Verwendung von festen sauren Kondensationsmitteln beträgt die Menge des festen Kondensationsmittels im allgemeinen 0,01 bis 1 l Schüttvolumen pro 1 Reaktorvolumen. Die festen sauren Kondensationsmittel können als solche oder auf einem Trägermaterial verwendet werden. Geeignete Trägermaterialien sind z.B. Aluminiumoxid, Kieselsäure, Aktivkohle.

Für die Veretherung wird das $C_4$-Kohlenwasserstoffgemisch mit dem primären Alkohol in Gegenwart des sauren Kondensationsmittels im allgemeinen bei Temperaturen von 20 bis 120°C, vorzugsweise 25 bis 100°C, insbesondere 30 bis 90°C umgesetzt.

Bei Verwendung von Ionenaustauschern in der Wasserstoffform als saure Kondensationsmittel wird die Veretherung mit besonderem Vorteil in der Weise durchgeführt, daß die Austrittstemperatur des Reaktionsgemisches aus der Reaktionszone für die Veretherung im Bereich von 25 bis 80°C, vorzugsweise 30 bis 70°C, insbesondere 30 bis 50°C liegt. Vorzugsweise werden Austrittstemperaturen angewendet, die tiefer liegen als die mittlere Temperatur in der Veretherungsstufe. Im allgemeinen wird in der Veretherungsreaktion das im $C_4$-Kohlenwasserstoffgemisch enthaltene Isobuten zu mindestens 90 %, vorzugsweise zu mindestens 95 %, insbesondere zu mindestens 96 % zum Alkyl-tert.-butylether umgesetzt. Es kann jedoch auch zweckmäßig sein, das Isobuten nur teilweise, z.B. zu 10 bis 90 %, vorzugsweise 20 bis 90 % zum Alkyl-tert.-butylether umzusetzen, falls z.B. durch eine gegebene Marktsituation, nur eine verringerte Menge an Alkyl-tert.-butylether anfallen soll.

Die erfindungsgemäße Veretherung kann bei Normaldruck erfolgen. Es ist jedoch zweckmäßig, einen geringen Überdruck z.B. Drücke von 1,01 bis 30 bar, insbesondere 2 bis 20 bar, anzuwenden. Das Isobuten und Butadien enthaltende $C_4$-Kohlenwasserstoffgemisch kann dabei je nach Druck und Temperatur für die Umsetzung flüssig oder gasförmig eingesetzt werden. Vorzugsweise werden flüssige Isobuten und Butadien enthaltende $C_4$-Kohlenwasserstoffgemische eingesetzt. Die Veretherung kann diskontinuierlich durchgeführt werden. Die Reaktionszeiten liegen bei diskontinuierlicher Arbeitsweise im allgemeinen zwischen 1 Minute und 5 Stunden. Vorzugsweise wird die Veretherung jedoch kontinuierlich durchgeführt, wobei das Verhältnis aus dem Volumen der Reaktionszone (in Volumeneinheiten) und dem Durchsatz in Volumeneinheiten je Stunde im allgemeinen 0,01 bis 5 Stunden, vorzugsweise 0,02 bis 1 Stunde, insbesondere 0,03 bis 1 Stunde, beträgt.

Das Molverhältnis von primärem $C_1$- bis $C_6$-Alkohol zu dem im $C_4$-Kohlenwasserstoffgemisch enthaltenen Isobuten beträgt bei der Veretherung im allgemeinen 100 : 1 bis 0,5 : 1, vorzugsweise 20 : 1 bis 0,7 : 1, inbesondere 4 : 1 bis 0,8 : 1.

Als Reaktortypen für die Veretherung eignen sich z.B. diskontinuierlich oder kontinuierlich betriebene Rührkessel, Rührkesselkaskaden, Wirbelschichten, Festbettreaktoren sowie Schlaufenreaktoren. Im allgemeinen werden Schlaufenreaktoren oder vorzugsweise Festbettreaktoren eingesetzt.

Das nach der Veretherung erhaltene Reaktionsgemisch wird anschließend durch ein- oder mehrstufige extraktive Destillation in

- einen die im selektiven Lösungsmittel schwerer als Butadien löslichen Kohlenwasserstoffe enthaltenden Strom,
- einen das Butadien enthaltenen Strom und
- einen den gebildeten Alkyl-tert.-butylether und die im selektiven Lösungsmittel leichter als Butadien löslichen Kohlenwasserstoffe enthaltenden Strom aufgetrennt.

Dabei kann es vorteilhaft sein, daß man aus dem aus der Veretherung erhaltenen Reaktionsgemisch darin gegebenenfalls enthaltenen nicht umgesetzten primären $C_1$- bis $C_6$-Alkohol vor der Auftrennung des Reaktionsgemisches in der nachgeschalteten Extraktivdestillation abtrennt. Dies ist z.B. von Vorteil, wenn der primäre Alkohol mit den Kohlenwasserstoffen Azeotrope bildet und gleichzeitig hohe Reinheiten gefordert sind. Die Abtrennung des primären Alkohols aus dem Reaktionsgemisch kann z.B. durch Destillation oder vorzugsweise durch eine Wasserwäsche, z.B. Absorption bzw. Flüssig-flüssig-Extraktion mit Wasser, erfolgen. Aus der durch die Wasserbehandlung erhaltenen Alkohol/Wasser-Mischung wird der primäre Alkohol zweckmäßig, z.B. durch Destillation zurückgewonnen. Der zurückgewonnene Alkohol wird zweckmäßig in die Veretherungsstufe zurückgeführt.

Das in die Extraktionsdestillation eingeführte Reaktionsgemisch aus der Veretherung, das die nicht umgesetzten $C_4$-Kohlenwasserstoffe, den gebildeten Alkyl-tert.-butylether und gegebenenfalls nicht umgesetzten primären $C_1$- bis $C_6$-Alkohol enthält, wird durch ein- oder mehrstufige, zweckmäßig ein- oder zweistufige Extraktivdestillation aufgetrennt, wobei die zweistufige Extraktivdestillation bevorzugt wird.

Beispielsweise wird die zweistufige Extraktivdestillation in der Weise durchgeführt, daß das aus der Veretherung erhaltene Reaktionsgemisch in zwei hintereinandergeschalteten Extraktivdestillationszonen zweckmäßig unter Verwendung des gleichen selektiven Lösungsmittels aufgetrennt wird, wobei in der ersten Extraktivdestillation - zweckmäßig als Kopfprodukt - ein die schwerer als Butadien löslichen Kohlenwasserstoffe enthaltender Strom, der im allgemeinen die Butane und die nicht umgesetzten Butene enthält, abgezogen wird. Gleichzeitig wird aus der ersten Extraktivdestillation ein Butadien, den gebildeten Alkyl-tert.-butylether und die leichter als Butadien löslichen Kohlenwasserstoffe wie Vinylacetylen, Butin-1 und Butadien-1,2 enthaltender Extrakt erhalten, der in einer Lösungsmittelwiedergewinnungszone, die beispielsweise als Ausgaser oder als Lösungsmittelabstreifer betrieben wird, vom selektiven Lösungsmittel befreit wird, wobei ein Gemisch aus Butadien, den leichter als Butadien löslichen Kohlenwasserstoffen und dem Alkyl-tert.-butylether erhalten wird. Das aus dem Extrakt erhaltene Gemisch wird einer zweiten Extraktivdestillation mit dem selektiven Lösungsmittel unterworfen, wobei das Butadien als Destillat und ein Extrakt erhalten werden, der den Alkyl-tert.-butylether und die leichter als Butadien löslichen Kohlenwasserstoffe enthält. In einer Lösungsmittelwiedergewinnungszone, z.B. in einem Ausgaser, wird der erhaltene Extrakt anschließend zweckmäßig vom selektiven Lösungsmittel befreit, wobei ein den Alkyl-tert.-butylether und die leichter als Butadien löslichen Kohlenwasserstoffe enthaltender Strom erhalten wird. Das wiedergewonnene selektive Lösungsmittel, das zweckmäßig am Sumpf der Lösungsmittelwiedergewinnungszone abgezogen wird, wird im allgemeinen in die Extraktivdestillation zurückgeführt.

Das abgezogene selektive Lösungsmittel kann weitgehend frei von Alkyl-tert.-butylether abgezogen werden. Es kann jedoch vorteilhaft sein, z.B. zur leichteren Einhaltung eines stationären Zustandes in der Extraktionsanlage, selektives Lösungsmittel aus der Lösungsmittelwiedergewinnungszone abzuziehen und zurückzuführen, welches noch Alkyl-tert.--butylether enthält. Im allgemeinen beträgt der Gehalt an Alkyl-tert.-butylether im zurückgeführten Lösungsmittel 1 bis 50 Gew.%, vorzugsweise 3 bis 30 Gew.%, bezogen auf die Mischung aus selektivem Lösungsmittel und Alkyl-tert.--butylether.

Der den Alkyl-tert.-butylether und die leichter als Butadien löslichen Kohlenwasserstoffe enthaltende Strom kann, gegebenenfalls nach Hydrierung, z.B. als Benzinzusatz verwendet werden oder weiterverarbeitet werden, beispielsweise um das darin enthaltene Vinylacetylen durch katalytische Hydrierung in Butadien umzuwandeln oder um das CH-acide Vinylacetylen mit metallorganischen Verbindungen, z.B. des Grignard-Typs, umzusetzen. Im allgemeinen wird man diesen Strom jedoch einer Destillation unterwerfen, wobei man zweckmäßig erhöhten Druck, z.B. Druck von 1,1 bis 10 bar, vorzugsweise 3 bis 6 bar anwendet. Dabei wird der Alkyl-tert.-butylether am Sumpf der Destillation erhalten, während am Kopf ein die leichter als Butadien löslichen Kohlenwasserstoffe, d.h. im allgemeinen Vinylacetylen, Butin-1 und Butadien-1,2, enthaltender Strom abgezogen wird. Um den Partialdruck der $C_4$-Acetylene (Vinylacetylen, Butin-1) im Kopfprodukt nicht zu hoch ansteigen zu lassen, gibt man zweckmäßig gesättigte und/oder einfach olefinisch ungesättigte Kohlenwasserstoffe zur Destillation, welche zusammen mit den $C_4$-Acetylenen als Kopfprodukt abgezogen werden und hierdurch für eine Verdünnung der $C_4$-Acetylene sorgen. Hierdurch läßt sich auch bei völliger oder teilweiser Abtrennung der Alkyl-tert.--butylether von den $C_4$-Acetylenen in der Destillationszone die Konzentration der $C_4$-Acetylene in einem genügend niedrigen Bereich halten, so daß die $C_4$-Acetylene gefahrlos gehandhabt werden können.

Die nachstehenden Beispiele dienen der weiteren Erläuterung der Erfindung:

Beispiel 1

Die Veretherung wird unter Verwendung eines $C_4$-Kohlenwasserstoffgemisches der folgenden Zusammensetzung durchgeführt:

| Isobutan | 2,5 Gew.% |
|---|---|
| n-Butan | 7,4 " |
| Buten-1 | 14,0 " |
| Isobuten | 20,0 " |
| trans-Buten-2 | 4,6 " |
| cis-Buten-2 | 3,5 " |
| Butadien-1,3 | 46,0 " |
| Butadien-1,2 | 0,3 " |
| Butin-1 | 0,3 " |
| Vinylacetylen | 1,2 " |
| $C_3$-Kohlenwasserstoffe | 0,1 " |
| $C_5$-Kohlenwasserstoffe | 0,1 " |

Eine Mischung von 10 kg je Stunde dieses $C_4$-Kohlenwasserstoffgemisches mit 1,1 kg je Stunde Methanol wird in ein rohrförmiges Reaktionsgefäß aus rostfreiem Stahl eingeleitet, in dem sich 2500 ml eines sulfonierten Polystyroldivinylbenzol-Harzes in der Wasserstofform (Lewatit SPC 118, Kornfraktion 0,5 bis 1 mm) befinden. In dem Reaktionsgefäß wird eine Temperatur von 60°C und ein Druck von 15 bar aufrechterhalten. Das nach der Veretherung erhaltene Reaktionsgemisch wird zur Entfernung und Rückgewinnung von nicht umgesetztem Methanol mit Wasser gewaschen.

Das Reaktionsprodukt aus der Veretherung wird nach der Wasserwäsche in eine Anlage zur Butadienextraktion eingeleitet, die mit N-Methylpyrrolidon (NMP) als selektives Lösungsmittel betrieben wird, wobei dem NMP kein Wasser zur Siedepunktserniedrigung und gleichzeitigen Selektivitätserhöhung zugesetzt wird. Anstelle des Wassergehaltes wird im NMP im stationären Betrieb der Butadienextraktionsanlage ein Gehalt an Methyl-tert.-butylether, dem Reaktionsprodukt der Veretherungsstufe, von etwa 9 Gew.%, bezogen auf die Mischung aus NMP und Methyl-tert.-butylether, aufrechterhalten.

In der Butadienextraktionsanlage sind zwei Extraktivdestillationen, die mit dem gleichen selektiven Lösungsmittel NMP betrieben werden, hintereinandergeschaltet. In der ersten Extraktivdestillation wird das Reaktionsprodukt aus der Veretherung in ein die Butane und Butene enthaltendes Destillat sowie einen Butadien-1,3, Vinylacetylen, Butin-1, Butadien-1,2 und den in der Veretherung gebildeten Methyl-tert.-butylether enthaltenden Extrakt aufgetrennt. Der Extrakt wird anschließend einer zweiten Extraktivdestillation unterworfen, in der als Destillat Butadien-1,3 erhalten wird, während der Extrakt Vinylacetylen, Butin-1, Butadien-1,2 und gebildeten Methyl-tert.-butylether enthält. Der erhaltene Extrakt wird einem Ausgaser zugeführt, aus dem ein Vinylacetylen, Butin-1, Butadien-1,2 und einen Teil des Methyl-tert.-butylethers enthaltender Strom erhalten wird. Am Boden des Ausgasers wird das entgaste selektive Lösungsmittel NMP, das einen Gehalt von 9 Gew.% Methyl-tert.-butylether aufweist, abgezogen und in die Extraktivdestillation zurückgeführt.

Das als Destillat der zweiten Extraktivdestillation erhaltene Butadien-1,3 wird anschließend zur Abtrennung von noch vorhandenen sehr geringen Mengen von $C_3$- und $C_5$-Kohlenwasserstoffen einer fraktionierten Destillation unterworfen.

Der aus dem Ausgaser erhaltene Strom aus Vinylacetylen, Butin-1, Butadien-1,2, Butadien-1,3 und $C_5$-Kohlenwasserstoffen und 92 Gew.% Methyl-tert.-butylether wird anschließend in einer Destillationskolonne, der gleichzeitig ein Butan/Buten-Strom zugeführt wird, in ein Kopfprodukt, welches Vinylacetylen, Butin-1 und Butadien-1,2 und als Verdünnungsmittel die Butane und Butene enthält, und Methyl-tert.-butylether, der am Boden abgezogen wird, aufgetrennt.

## Beispiel 2

Man verfährt wie im Beispiel 1 beschrieben, wobei jedoch in der Veretherungsstufe anstelle von Methanol 3,8 kg je Stunde n-Propanol eingesetzt werden. Dabei wird auf eine Wasserwäsche des Reaktionsproduktes aus der Veretherung zur Abtrennung von nicht umgesetztem n-Propanol verzichtet, da n-Propanol im Gegensatz zum Methanol praktisch keine störenden Azeotrope mit den $C_4$-Kohlenwasserstoffen bildet.

Aus dem der zweiten Extraktivdestillation nachgeschalteten Ausgaser wird ein Strom abgezogen, der gebildeten n-Propyl--tert.-butylether, n-Propanol, Vinylacetylen, Butin-1 und Butadien-1,2 enthält. Bei der nachfolgenden Destillation dieses Stromes unter Zuführung eines Butan/Buten-Gemisches wird als Sumpfprodukt eine Mischung aus dem n-Propyl--tert.-butylether und n-Propanol erhalten.

Diese Mischung kann ohne Abtrennung des n-Propanols zur Herstellung von hochreinem Isobuten durch Spaltung des n-Propyl-tert.-butylethers an einem sauren Katalysator verwendet werden.

Patentansprüche

1. Verfahren zur gemeinsamen Herstellung von Alkyl-tert.- -butylether und Gewinnung von Butadien mit Hilfe eines selektiven Lösungsmittels aus einem $C_4$-Kohlenwasserstoffgemisch, welches Butadien, Isobuten, im selektiven Lösungsmittel leichter als Butadien lösliche Kohlenwasserstoffe und im selektiven Lösungsmittel schwerer als Butadien lösliche Kohlenwasserstoffe enthält, dadurch gekennzeichnet, daß man

a) das $C_4$-Kohlenwasserstoffgemisch in Gegenwart eines sauren Katalysators mit einem primären $C_1$- bis $C_6$-Alkohol reagieren läßt, wobei das Isobuten ganz oder teilweise zum Alkyl-tert.- -butylether umgesetzt wird, und

b) das erhaltene Reaktionsgemisch anschließend durch ein- oder mehrstufige Extraktivdestillation in

- einen die im selektiven Lösungsmittel schwerer als Butadien löslichen Kohlenwasserstoffe enthaltenden Strom,
- einen das Butadien enthaltenden Strom und
- einen den gebildeten Alkyl-tert.-butylether und die im selektiven Lösungsmittel leichter als Butadien löslichen Kohlenwasserstoffe enthaltenden Strom auftrennt.

2. Verfahren nach Anspruch 1, dadurch gekennzeichnet, daß als selektives Lösungsmittel ein N-alkylsubstituiertes niederes aliphatisches Säureamid, ein N-alkylsubstituiertes alicyclisches Säureamid mit 5 Ringgliedern, Acetonitril, Methoxypropionitril, Butyrolacton, N-Formylmorpholin oder Furfurol verwendet wird.

3. Verfahren nach Anspruch 1 bis 2, dadurch gekennzeichnet, daß das in der Extraktivdestillation verwendete selektive Lösungsmittel 1 bis 90 Gew.% gebildeten Alkyl-tert.-butylether, bezogen auf die Mischung aus selektivem Lösungsmittel und gebildetem Alkyl-tert.-butylether, enthält.

4. Verfahren nach Anspruch 1 bis 3, dadurch gekennzeichnet, daß das aus der Veretherung erhaltene Reaktionsgemisch in zwei hintereinandergeschalteten Extraktivdestillationszonen unter Verwendung des gleichen selektiven Lösungsmittels aufgetrennt wird, wobei

a) in der ersten Extraktivdestillations ein die schwerer als Butadien löslichen Kohlenwasserstoffe enthaltendes Destillat (Raffinat) und ein Butadien, die leichter als Butadien löslichen Kohlenwasserstoffe und den gebildeten Alkyl-tert.-butylether enthaltender Extrakt erhalten werden,

b) der Extrakt vom selektiven Lösungsmittel befreit wird, wobei ein Gemisch aus Butadien, den leichter als Butadien löslichen Kohlenwasserstoffen und dem Alkyl-tert.-butylether erhalten wird, und

c) das aus dem Extrakt erhaltene Gemisch in der zweiten Extraktivdestillation in einen das Butadien enthaltenden Strom und einen den Alkyl--tert.-butylether und die leichter als Butadien löslichen Kohlenwasserstoffe enthaltenden Strom aufgetrennt wird.

5. Verfahren nach Anspruch 4, dadurch gekennzeichnet, daß der in der zweiten Extraktivdestillation erhaltene den Alkyl-tert.-butylether und die leichter als Butadien löslichen Kohlenwasserstoffe enthaltende Strom durch Destillation in einen den Alkyl-tert.--butylether und einen die leichter als Butadien löslichen Kohlenwasserstoffe enthaltenden Strom aufgetrennt wird.

6. Verfahren nach Anspruch 1 bis 5, dadurch gekennzeichnet, daß man aus dem aus der Veretherung erhaltenen Reaktionsgemisch darin enthaltenen nicht umgesetzten primären $C_1$- bis $C_6$-Alkohol vor der Auftrennung des Reaktionsgemisches in der nachgeschalteten Extraktivdestillation abtrennt.

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | EP - A - 0 017 116 (BASF) * Seite 4, Zeile 10 - Seite 6, Zeile 7; Seiten 7-9; Beispiel 1 * & DE - A - 2 911 393 | 1-5 |
| D | FR - A - 2 272 056 (SNAM PROGETTI) * Seite 2, Zeile 30 - Seite 4, Zeile 26; Beispiel; Ansprüche 1-4,6,7,9,11,12 * & DE - A - 2 521 965 | 1,2,4, 5 |
| A | FR - A - 2 312 483 (ERAP) * Seite 2, Zeile 36 - Seite 5, Zeile 20; Seite 4, Zeile 32 und Seite 5, Anspruch 6 * | |
| A | GB - A - 2 047 706 (IFP) * Anspruch 1 * | |

### KLASSIFIKATION DER ANMELDUNG (Int. Cl.³)

C 07 C 11/167
7/148
7/08
41/06
41/42
43/04

### RECHERCHIERTE SACHGEBIETE (Int. Cl.³)

C 07 C 7/00
7/08
7/148
41/00
41/05
41/06
41/34
41/42

### KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 25-01-1982 | LION |

EPA form 1503.1 06.78